# EUROPEAN PATENT APPLICATION

(11) **EP 4 431 152 A1**
(43) Date of publication of application: **18.09.2024**
(21) Application number: 23162694.6
(22) Date of filing: 17.03.2023
(51) Int. Cl.: A61P 35/00, C07K 16/28, A61K 39/00

(54) **COMBINATIONS OF AN ANTI-INTERLEUKIN-6 RECEPTOR ANTIBODY, A PLATINUM-BASED ANTINEOPLASTIC DRUG, AND A TAXANE**

(71) Applicant: King Faisal Specialist Hospital & Research Centre, Riyadh, 11211 (SA)
(72) Inventor: ABOUSSEKHRA, Abdelilah, 11211 Riyadh (SA); AL-TWEIGERI, Taher, 11211 Riyadh (SA); ALRAOUJI, Noura, 11211 Riyadh (SA)
(74) Representative: Engelhard, Markus

(57) **Abstract**

The present invention relates to combinations of an anti-interleukin-6 receptor antibody, a platinum-based antineoplastic drug, and a taxane, and their uses in a method of treatment of breast cancer.

## Description

### FIELD OF THE INVENTION

The present invention relates to combinations of an anti-interleukin-6 receptor antibody, a platinum-based antineoplastic drug, and a taxane, and their uses in a method of treatment of breast cancer.

### BACKGROUND OF THE INVENTION

Breast cancer, the most common type of cancer, is the leading cause of cancer-related deaths among women worldwide. Locally advanced breast cancer continues to be a serious health problem with adverse outcome despite all the revolutionary advancements made in cancer therapy and the introduction of precision medicine.

In recent decades the survival rate of breast cancer patients has improved especially in developed countries due to advances in treatment and early screening. However, recurrence and metastasis remain a major problem and are the main cause of death in breast cancer patients. Previous studies show that approximately 75% of primary breast tumors have already spread at the time of diagnosis and almost 30% of patients who are free of disease after initial local and regional treatments develop disease recurrence.

Cancer immunotherapy via remodeling the host immune system to eradicate tumor cells has recently become an area of extensive research. Interleukin-6 (IL-6) is a pleiotropic pro-inflammatory cytokine secreted by a variety of cell types which has a vital role in host defense immunity and acute stress. IL-6 acts through a specific receptor (IL-6R), present in both soluble and membrane-bound forms.

Triple-negative breast cancer (TNBC), an aggressive subgroup of human breast cancer, which is characterized as estrogen receptor (ER) negative, progesterone receptor (PR) negative, and human epidermal growth factor receptor 2 (HER2) negative, accounts for approximately 15%-20% of newly diagnosed breast cancer worldwide. Because TNBC lacks the targetable receptors that are expressed in other types of breast tumor, treating this notoriously aggressive type of breast cancer is challenging. Conventional chemotherapy remains the primary treatment option for patients with early and advanced-stage TNBC, with anthracycline and taxane-based chemotherapy being the mainstay of the therapy. In the past few years, neo-adjuvant chemotherapy with platinum-based agents has been reported to produce therapeutic benefits in a portion of the patients with chemo-sensitive TNBC. Nevertheless, despite comprehensive and aggressive management, over 50% of TNBC patients (stage I-III) recur, and more than 37% of those patients succumb within 5 years. Thus, novel effective therapies for TNBC are urgently needed, and have become one of the main focuses of breast cancer research. Like other types of breast cancer, TNBC cells were also shown to secrete high levels of IL-6.

Tocilizumab (Actemra) is a humanized anti-human IL-6R antibody, which efficiently inhibits the IL-6 signaling pathway through blocking both forms of the IL-6R. Tocilizumab is approved by US Food and Drug Administration (FDA) to treat several IL-6-dependent chronic inflammatory diseases, such as rheumatoid arthritis and cytokine released syndrome.

### SUMMARY OF THE INVENTION

The scope of the invention is defined by the appended set of claims. In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

One aspect of the present invention is a combination of an anti-interleukin-6 receptor antibody, a platinum-based antineoplastic drug, and a taxane, for use in a method of treatment of breast cancer.

In one embodiment, said anti-interleukin-6 receptor antibody is a humanized anti-interleukin-6 receptor antibody; preferably said humanized anti-interleukin-6 receptor antibody is selected from: tocilizumab and a tocilizumab biosimilar; more preferably said humanized anti-interleukin-6 receptor antibody is tocilizumab; and
said platinum-based antineoplastic drug is selected from one or more of: cisplatin and carboplatin; preferably said platinum-based antineoplastic drug is cisplatin; and
said taxane is selected from one or more of: docetaxel, paclitaxel, and nab-paclitaxel; preferably said taxane is docetaxel.

In one embodiment, said combination is administered to a patient in need thereof; preferably wherein said patient is a mammal; more preferably wherein said patient is a human.

In one embodiment, said combination is administered in a sequential manner, wherein said sequential manner comprises a dosing regimen selected from: administering a dose of said anti-interleukin-6 receptor antibody, followed by administering a dose of said platinum-based antineoplastic drug and a dose of said taxane; or administering a dose of said anti-interleukin-6 receptor antibody, followed by administering a dose of said platinum-based antineoplastic drug, followed by a dose of said taxane; or administering a dose of said anti-interleukin-6 receptor antibody, followed by administering a dose of said taxane, followed by a dose of said platinum-based antineoplastic drug;
or administering a dose of said platinum-based antineoplastic drug, followed by administering a dose of said anti-interleukin-6 receptor antibody and a dose of said taxane; or administering a dose of said platinum-based antineoplastic drug, followed by administering a dose of said anti-interleukin-6 receptor antibody, followed by a dose of said taxane; or administering a dose of said platinum-based antineoplastic drug, followed by administering a dose of said taxane, followed by a dose of said anti-interleukin-6 receptor antibody;
or administering a dose of said taxane, followed by administering a dose of said anti-interleukin-6 receptor antibody and a dose of said platinum-based antineoplastic drug; or administering a dose of said taxane, followed by administering a dose of said anti-interleukin-6 receptor antibody, followed by a dose of said platinum-based antineoplastic drug; or administering a dose of said taxane, followed by administering a dose of said platinum-based antineoplastic drug, followed by a dose of said anti-interleukin-6 receptor antibody;
preferably administering a dose of said anti-interleukin-6 receptor antibody, followed by administering a dose of said platinum-based antineoplastic drug and a dose of said taxane;
or said combination is administered in a simultaneous manner, wherein said simultaneous manner comprises administering a dose of said anti-interleukin-6 receptor antibody, a dose of said platinum-based antineoplastic drug, and a dose of said taxane at the same time.

In one embodiment, said method of treatment of breast cancer comprises
(1) administering a dose of said anti-interleukin-6 receptor antibody to a patient in need, on a first day of treatment;
(2) administering a dose of said platinum-based antineoplastic drug and a dose of said taxane to the patient in need, on days 2-8 of treatment, preferably on days 2-4 of treatment, more preferably on day 2 of treatment; and optionally
(3) administering a dose of said platinum-based antineoplastic drug and a dose of said taxane to the patient in need, in a periodical manner every 2-8 weeks, preferably every 2-6 weeks, more preferably every 4 weeks.

In a preferred embodiment, said administering a dose of said platinum-based antineoplastic drug and a dose of said taxane of steps (2) and (3) is performed for a total of 4 to 7 cycles; preferably for a total of 6 cycles.

In one embodiment, said anti-interleukin-6 receptor antibody is administered to said patient at a dosage of said anti-interleukin-6 receptor antibody in the range of 1 to 25 mg/kg; preferably in the range of 1 to 15 mg/kg; more preferably in the range of 1 to 12 mg/kg; most preferably in the range of 4 to 8 mg/kg.

In one embodiment, said platinum-based antineoplastic drug is administered to said patient at a dosage of said platinum-based antineoplastic drug in the range of 1 to 250 mg/m²; preferably in the range of 1 to 180 mg/m²; more preferably in the range of 1 to 120 mg/m²; most preferably in the range of 30 to 60 mg/m².

In one embodiment, said taxane is administered to said patient at a dosage of said taxane in the range of 1 to 250 mg/m²; preferably in the range of 1 to 180 mg/m²; more preferably in the range of 1 to 120 mg/m²; most preferably in the range of 30 to 60 mg/m².

In one embodiment, said breast cancer is selected from: triple-negative breast cancer, locally advanced non-inflammatory breast cancer, invasive ductal carcinoma, inflammatory breast cancer, metastatic breast cancer, medullary carcinoma, tubular carcinoma, mucinous carcinoma, adenoid cystic carcinoma of the breast, metaplastic breast cancer, basal type breast cancer, or papillary breast cancer.

In one embodiment, the cells of said breast cancer contain mutations in the BRCA1 and/or BRCA2 gene.

In one embodiment, cells of said breast cancer are characterized by being estrogen receptor negative, or progesterone receptor negative, or human epidermal growth factor receptor 2 negative, or any possible combination thereof; preferably wherein cells of the cancer are characterized by a combination thereof; even more preferably wherein cells of the cancer are characterized by being estrogen receptor negative, progesterone receptor negative, and human epidermal growth factor receptor 2 negative, also called triple-negative breast cancer.

In one embodiment, said anti-interleukin-6 receptor antibody is formulated and administered as an injection intravenously or subcutaneously.

In one embodiment, said platinum-based antineoplastic drug is formulated and administered as an injection intravenously.

In one embodiment, said taxane is formulated and administered as an injection intravenously.

In one embodiment, said anti-interleukin-6 receptor antibody is formulated and administered as an injection intravenously or subcutaneously; said platinum-based antineoplastic drug is formulated and administered as an injection intravenously; and said taxane is formulated and administered as an injection intravenously.

In one embodiment, said anti-interleukin-6 receptor antibody and/or said platinum-based antineoplastic drug and/or said taxane are administered in conjunction with further agent(s) or drug(s), such as granulocyte colony-stimulating factor.

In one embodiment, said anti-interleukin-6 receptor antibody is tocilizumab, said platinum-based antineoplastic drug is cisplatin, and said taxane is docetaxel.

In a further aspect, the present invention also relates to a kit for the preparation of an infusion solution for the administration of a combination of an anti-interleukin-6 receptor antibody, a platinum-based antineoplastic drug, and a taxane, said combination being as defined herein, said kit comprising a first container containing a composition comprising an anti-interleukin-6 receptor antibody, and a second container containing a composition comprising a pharmaceutically acceptable diluent; and/or
comprising a container containing a composition comprising a platinum-based antineoplastic drug, or a pharmaceutically acceptable salt thereof, and another container containing a composition comprising a pharmaceutically acceptable diluent; and/or
comprising a container containing a composition comprising a taxane, or a pharmaceutically acceptable salt thereof, and another container containing a composition comprising a pharmaceutically acceptable diluent.

In one embodiment of said kit, said containers of the kit are selected from vials, bottles, pre-filled syringes, ampoules, infusion bags.

In one embodiment of said kit, the containers contain pre-mixed solutions selected from: said anti-interleukin-6 receptor antibody pre-mixed with said platinum-based antineoplastic drug, or a pharmaceutically acceptable salt thereof; said anti-interleukin-6 receptor antibody pre-mixed with said taxane, or a pharmaceutically acceptable salt thereof; said anti-interleukin-6 receptor antibody pre-mixed with said platinum-based antineoplastic drug, or a pharmaceutically acceptable salt thereof and said taxane, or a pharmaceutically acceptable salt thereof; and said platinum-based antineoplastic drug, or a pharmaceutically acceptable salt thereof pre-mixed with said taxane, or a pharmaceutically acceptable salt thereof.

In one embodiment of said kit, said anti-interleukin-6 receptor antibody is provided as an injection concentrate;
said platinum-based antineoplastic drug is provided in a form selected from: an injection concentrate, a pre-diluted solution, and a lyophilized powder for reconstitution; and
said taxane is provided in a form selected from: an injection concentrate, a pre-diluted solution, and a lyophilized powder for reconstitution.

Another aspect of the present application relates to a method of treating a breast cancer in a patient, wherein said method comprises administering a combination of an anti-interleukin-6 receptor antibody, a platinum-based antineoplastic drug, and a taxane, to a patient in need thereof; preferably wherein said patient is a mammal; more preferably wherein said patient is a human.

According to this aspect, the breast cancer, the patient, the combination and the method of treatment are as defined herein.

Another aspect of the present application is a use of a combination of an anti-interleukin-6 receptor antibody, a platinum-based antineoplastic drug, and a taxane, for the manufacture of a medicament for treatment of a breast cancer in a patient, preferably wherein said patient is a mammal; more preferably wherein said patient is a human.

According to this aspect, the breast cancer, the patient, and the method of treatment are as defined herein.

### DETAILED DESCRIPTION

The term "triple-negative breast cancer" (TNBC), as used herein, refers to a type of breast cancer wherein the cells are characterized as by being estrogen receptor negative, progesterone receptor negative, and human epidermal growth factor receptor 2 negative.

The term "estrogen receptor negative" (ER negative), as used herein, refers to the respective hormone receptor status. The hormone receptor status was considered negative if ≤5% of the tumor cells were stained during immunohistochemistry when using a stain targeting the estrogen receptor.

The term "progesterone receptor negative" (PR negative), as used herein, refers to the respective hormone receptor status. The hormone receptor status was considered negative if ≤5% of the tumor cells were stained during immunohistochemistry when using a stain targeting the progesterone receptor.

The term "human epidermal growth factor receptor 2 negative" (HER2 negative), as used herein, refers to the respective hormone receptor status. The hormone receptor status was considered negative if ≤9% of the tumor cells were stained during immunohistochemistry when using a stain targeting the human epidermal growth factor receptor 2.

The term "combination", as used herein, refers to the result of an act of combining entities, materials, compounds, or compositions in a spatial and/or temporal manner. In such combination, the combined entities, materials, compounds, or compositions can be administered together in a single composition or separately as separate entities, albeit at the same time, or one immediately after the other. Alternatively, in such combinations, the combined entities, materials, compounds, or compositions can be administered in a temporally separate manner, with intervals between the administration of the separate individual entities, materials, compounds, or compositions. A combination can also mean the joint use of the active ingredients in a method of treatment to achieve a beneficial effect, especially a synergistic therapeutic effect. For this joint use, the active ingredients can be administered together at the same time or in the form of mixed composition. Alternatively, a combination can also mean a separate, temporally distinct administration of the respective active ingredient in a defined dosing regimen, such defined dosing regimen comprising, however, the separate administration of all the ingredients that form part of the combination.

The term "anti-interleukin-6 receptor antibody", as used herein, refers to antibody raised against the interleukin-6 receptor (IL-6R).

The term "taxane", as used herein, refers to a member of the taxane family of medication. Taxanes are a class of diterpenes and feature a taxadiene core.

The term "platinum-based antineoplastic drug", as used herein, refers to a chemotherapeutic agents used to treat cancer. Platinum-based antineoplastic drugs are coordination complexes of platinum.

The term "tocilizumab", as used herein, refers to a humanized monoclonal antibody against the interleukin-6 receptor (IL-6R).

The term "docetaxel", as used herein, refers to a pharmaceutical compound with the chemical formula C₄₃H₅₃NO₁₄ that belongs to the chemotherapy drug class of taxanes.

The term "paclitaxel" as used herein, refers to a pharmaceutical compound with the chemical formula C₄₇H₅₁NO₁₄ that belongs to the chemotherapy drug class of taxanes.

The term "nab-paclitaxel" as used herein, refers to an injectable formulation of paclitaxel. In this formulation, paclitaxel is bonded to albumin as a delivery vehicle.

The term "cisplatin", as used herein, refers to a pharmaceutical compound of the platinum-based antineoplastic family of medications. Cisplatin is the square planar coordination complex with the chemical formula cis-[Pt(NH₃)₂Cl₂].

The term "carboplatin", as used herein, refers to a pharmaceutical compound of the platinum-based antineoplastic family of medications.

The term "administration", as used herein, refers to the application of a compound to a patient (either human or animal) having or suspected of having breast cancer. Said application can be performed as an injection intravenously or subcutaneously.

The term "simultaneous administration", as used herein, refers to the joint administration of active ingredients at the same time.

The term "administration in a sequential manner", as used herein, refers to the administration of active ingredients one after another during treatment.

The term "mg/m²", as used herein, refers to the amount (mass) of an active ingredient used per body surface area. The body surface area is the measured or calculated surface area of the human body. Depending on the type of medication, the body surface area is better indicator of metabolic mass than body weight because it is less affected by abnormal adipose mass.

The term "invasive ductal carcinoma", as used herein, refers to an invasive cancer where abnormal cancer cells that began forming in the milk ducts have spread beyond the ducts into other parts of the breast tissue. Invasive cancer cells can also spread to other parts of the body. It is also sometimes called infiltrative ductal carcinoma.

The term "inflammatory breast cancer", as used herein, refers to an aggressive and fast-growing breast cancer in which cancer cells infiltrate the skin and lymph vessels of the breast. It often produces no distinct tumor or lump that can be felt and isolated within the breast. But when the lymph vessels become blocked by the breast cancer cells, symptoms begin to appear.

The term "metastatic breast cancer", as used herein, refers to a cancer that has spread to other parts of the body. This usually includes the lungs, liver, bones or brain.

The term "medullary carcinoma", as used herein, refers to an invasive carcinoma, as a part of TNBC.

The term "tubular carcinoma", as used herein, refers to an invasive carcinoma, as a part of TNBC.

The term "mucinous carcinoma", as used herein, refers to an invasive carcinoma which starts in the main cells of mucus, called mucin.

The term "adenoid cystic carcinoma of the breast", as used herein, refers to an invasive carcinoma, as an aggressive type of TNBC.

The term "metaplastic breast cancer", as used herein, refers to an invasive carcinoma, as an aggressive type of TNBC.

The term "basal type breast cancer", as used herein, refers to a unique type of TNBC with particular genetic changes in the cells. The cells produce large amounts of cytokeratin 5/6.

The term "papillary breast cancer", as used herein, refers to an invasive carcinoma with benign behavior.

The term "locally advanced non-inflammatory breast cancer", as used herein, refers to breast cancer including those with stage IIIA-C breast tumors with metastases to ipsilateral axillary or internal mammary lymph nodes, or with direct extension into chest wall or through skin, without distant metastases.

The term "residual cancer burden" (RCB), as used herein, refers to a measure for the prognostic of outcomes across breast cancer subtypes. Class o is defined as no residual carcinoma in both breast and axilla. Class +1 indicates minimal residual disease (in breast and/or axilla). Class +2 indicates moderates residual disease in breast and/or axilla. Class +3 indicates severe residual disease in breast and/or axilla.

The term "pathological complete response" (pCR), as used herein, refers to complete absence of viable invasive tumor cells in the breast and axillary nodes, including surgical margins without ductal carcinoma in situ.

The terms "BRCA1" and "BRCA2", as used herein, refer to the human tumor suppressor genes "breast cancer gene 1" and "breast cancer gene 2", respectively. People with inherited harmful mutations in one of these genes have increased risks of several cancers-most notably breast and ovarian cancer, but also several additional types of cancer.

The term "kit" as used herein, refers to a package, a box, a carton, a bag, or a blister, containing one or more different compounds and/or their respective diluents necessary for the administration of said compounds.

The term "pharmaceutically acceptable diluent", as used herein, refers to a solution suitable for dilution or reconstitution of the respective active ingredients. Pharmaceutically acceptable diluents are well tolerated when applied to a patient and may be used to adjust the concentration of active ingredients to the desired range for application. When provided in a kit, the composition of pharmaceutically acceptable diluents may be different for each active ingredient that is to be diluted. The composition of the diluents depends, for example, on the solubility of the respective active ingredient in different solvents.

In one embodiment, the term "patient" relates to a human or an animal, preferably a human.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures, wherein

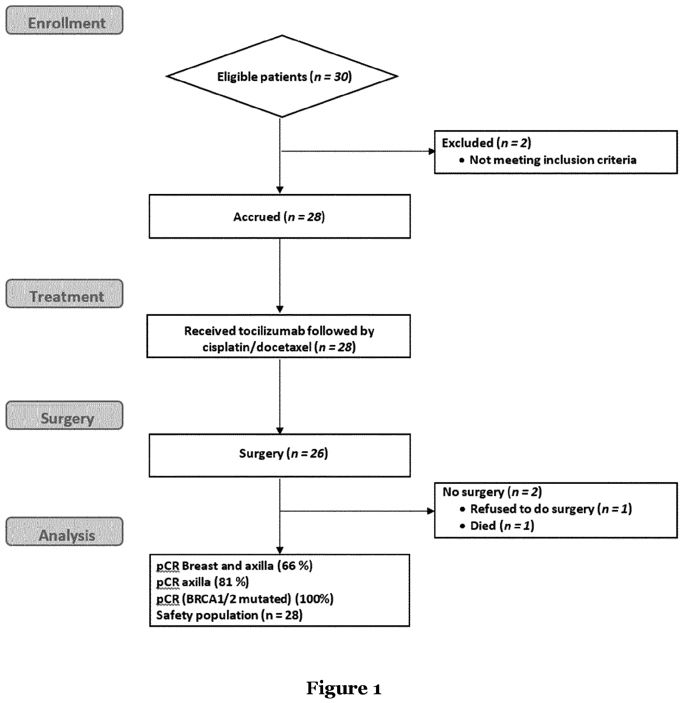

### Figure 1 shows: Consolidated Standards of Reporting Trials flow diagram.

In the following, reference is made to the examples, which are given to illustrate, not to limit the present invention.

### EXAMPLES

The following example demonstrates the novel use of the sequential administration of an anti-interleukin-6 receptor antibody, a platinum-based antineoplastic drug, and a taxane for treatment of breast cancer. Exemplified by the sequential administration of tocilizumab followed by cisplatin/docetaxel in the treatment of tiple-negative breast cancer (TNBC), the inventors describe how TNBC patients benefit from the applied dosing regimen. Without wishing to be bound by any theory, the inventors surmise that the clinical response to the dosing regimen, as exemplified by the effective treatment of TNBC, can be extrapolated to other forms of breast cancer.

### Example 1:

### Materials and methods

### Eligibility Criteria:

Female subjects with histological proven unilateral triple negative locally advanced non-inflammatory breast cancer (T2 > 2 cm, T3 or T4, N0-N3, M0) stages IIB and III were enrolled in this study. Patients were eligible if they were at least 18 years of age, had Eastern Cooperative Oncology Group (ECOG) performance status <2, and were to be neither pregnant nor nursing. Furthermore, all patients required to have a bidimensional measurable lesion, and biologic profile compatible with treatment adequate bone marrow reserve (ANC ≥ 1.5 × 10, platelet count ≥100 × 10, and hemoglobin level ≥100 g). Hepatic and renal functions were confirmed by pre-study examination (bilirubin level <1.5 the upper normal limit (UNL), AST and or ALT < 3 × UNL, prothrombin time <1.5 times control, creatinine clearance ≥60 ml min) and normal left ventricular ejection fraction (LVEF) and normal QTc on EKG. Patients were excluded from participation if there was evidence of inflammatory breast cancer; presence of metastasis; or prior malignancies; preexisting pulmonary; and/or cardiac disease or psychiatric illness.

### Overview of study design and approval:

This trial is a prospective single arm Phase I/II study, which investigated the pathological complete response (pCR) rate of neoadjuvant tocilizumab followed by cisplatin/ docetaxel. All eligible patients signed a written informed consent form, the study was approved by appropriate ethical review board at King Faisal Specialist Hospital and Research Center (RAC# 2181156) and Saudi FDA (SCTR No 19111002).

### Study Procedures:

This study has involved 30 patients from King Faisal Specialist Hospital & Research Centre, Riyadh. Patients were selected based on inclusion/exclusion criteria. Treatment was performed with tocilizumab 8 mg/kg administered intravenously on day 1 followed by cisplatin / docetaxel (60 mg/60 mg /m²) on day 2 every 4 weeks for a total of 6 cycles. Complete blood count was determined on day one of each cycle. Treatment was permitted only when white blood cell count was ≥3.0 × 109, absolute neutrophil count was ≥1.5 × 109, and platelet count was ≥100 × 109; if these values were not reached, treatment was delayed until recovery. All drugs were reduced by 25% in case of febrile neutropenia requiring hospitalization and/or IV antibiotics, grade 4 thrombocytopenia lasting more than 3 days and/ or associated with bleeding, or grade 4 neutropenia lasting more than 7 days. In case of grade 3 and grade 4 non-hematologic toxicities (except nausea/vomiting and alopecia), all drugs were reduced by 25% and 50%, respectively. The protocol permitted the use of granulocyte colony-stimulating factor (G-CSF) as prophylaxis after episodes of febrile neutropenia or prolonged neutropenia. Patients with hypersensitivity reactions received supportive measures and were treated again with docetaxel at a slower rate of infusion. Patients proceeded to surgery within four weeks after the last cycle of treatment. The decision to proceed to mastectomy or breast-conserving surgery (BCS) was at the discretion of the surgeon, according to best practice standard and the preference of each patient. Patients who underwent BCS received radiotherapy. Postmastectomy radiotherapy was also recommended for patients with at least clinical stage III disease or clinical tumor size ≥5 cm at diagnosis, or pathologic involvement of ≥4 axillary lymph nodes.

### Assessment of endpoints:

Initial assessment of patients was carried out within 3 weeks before study entry and included medical history, performance status, weight, height, and vital signs; physical examination, complete blood picture (CBC) with differential, platelet count, and renal and liver function tests were obtained within 2 weeks of registration. All patients were evaluated with bilateral mammogram, mammary ultrasound, bone scan, computed tomography of chest, abdomen, and pelvis or PETCT scan ± MRI if indicated to rule out distant metastasis. Left ventricular ejection fraction by multigated acquisition scan or by echocardiogram ≥55% was determined within 3 weeks of registration if indicated. All patients had to undergo tru-cut biopsy; surgical specimens were evaluated by a board-certified pathologist; the diagnosis of invasive breast cancer was confirmed. Assessment for grade, lympho-vascular invasion, Ki-67 labelling index, tumor infiltrating lymphocyte by the international tumor infiltrating lymphocyte (TILs) working group, TILs were scored on binary scale of ≤30% or >30%, estrogen and progesterone receptors and HER2 were determined on pretreatment biopsy by immunohistochemistry (IHC). Hormone receptor status was considered negative if ≤5% of tumor cells were stained for ER and/or PR. HER2 status was determined by immunohistochemistry (IHC) and confirmed with either chromogenic or fluorescent in situ hybridization if IHC equivocal (2+), FISH ratio of >2 was considered positive. Clinical response was assessed by physical examination using a caliper before each treatment cycle and defined as complete response, partial response, and progressive disease according to Response Evaluation Criteria in Solid Tumors. A pCR was defined as complete absence of viable invasive tumor cells in the breast and axillary nodes, including surgical margins without ductal carcinoma in situ (ypTo/ypNo). Toxicities were graded according to the National Cancer Institute Common Toxicity Criteria version 3.0.

### Results

### Enrollment and patient characteristics:

In the present study, a total of 30 TNBC patients, with stage IIb - III, were enrolled between January 2019 and July 2022. Two patients were found to be ineligible (one had metaplastic carcinoma and one presented signet ring carcinoma), 28 patients completed the study protocol, but only 26 patients underwent definitive surgery (while one patient has declined surgery and is still on follow up, the second one died with a cause not related to the study protocol). Patient's characteristics, disease status, and treatment variables are summarized in Table 1. The median age on inclusion was 42 years (25-65 years). Most of the patients presented tumors with high stage (23% clinical stage IIB, 77% stage III), and high grade (80% grade III). Patients with nodal involvement at baseline represented 67%. Interestingly, deleterious mutations in the BRCA1/2 genes, were detected in 8 out of 30 patients (27%). Eligible patients were treated intravenously with tocilizumab 8 mg /kg on day 1 followed by cisplatin / docetaxel (60 mg/60 mg /m²) on day 2 every 4 weeks for a total of 6 cycles.

A standard of reporting trials diagram is shown in Figure 1.

Following neoadjuvant treatment, surgery with lumpectomy (17% of patients) or mastectomy (77% of patients) was applied. All patients who had risk of relapse had adjuvant radiotherapy as per institutional guidelines.

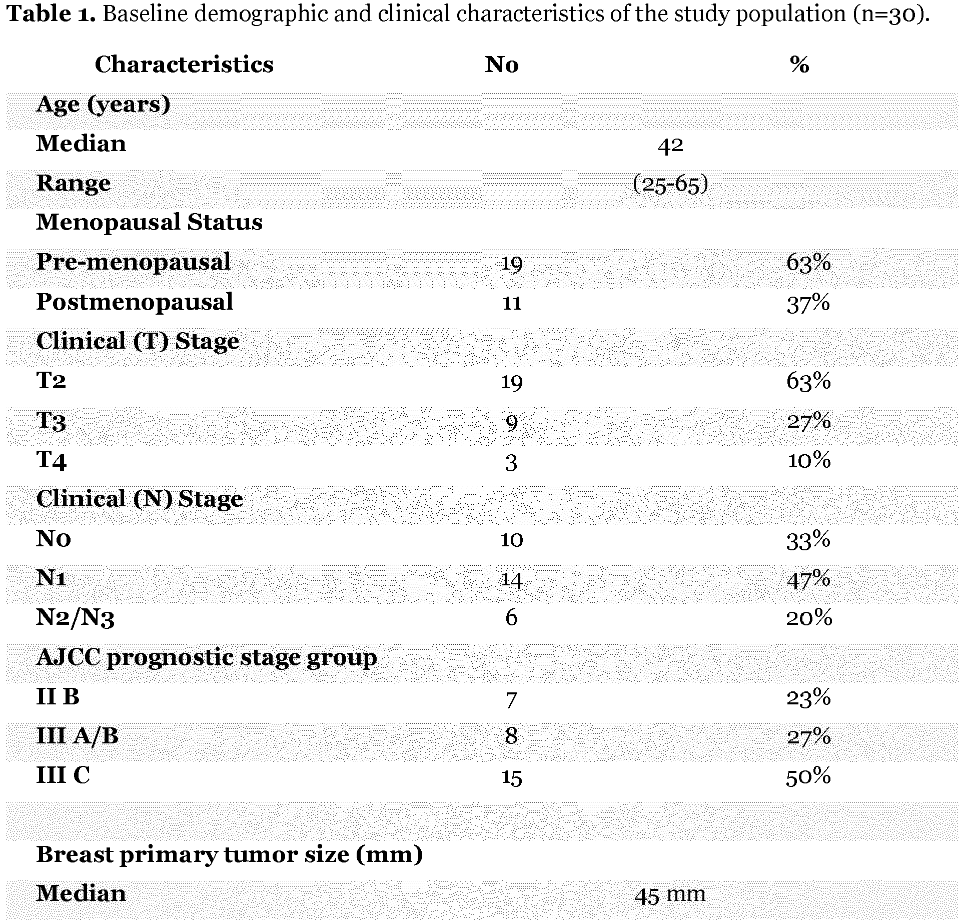

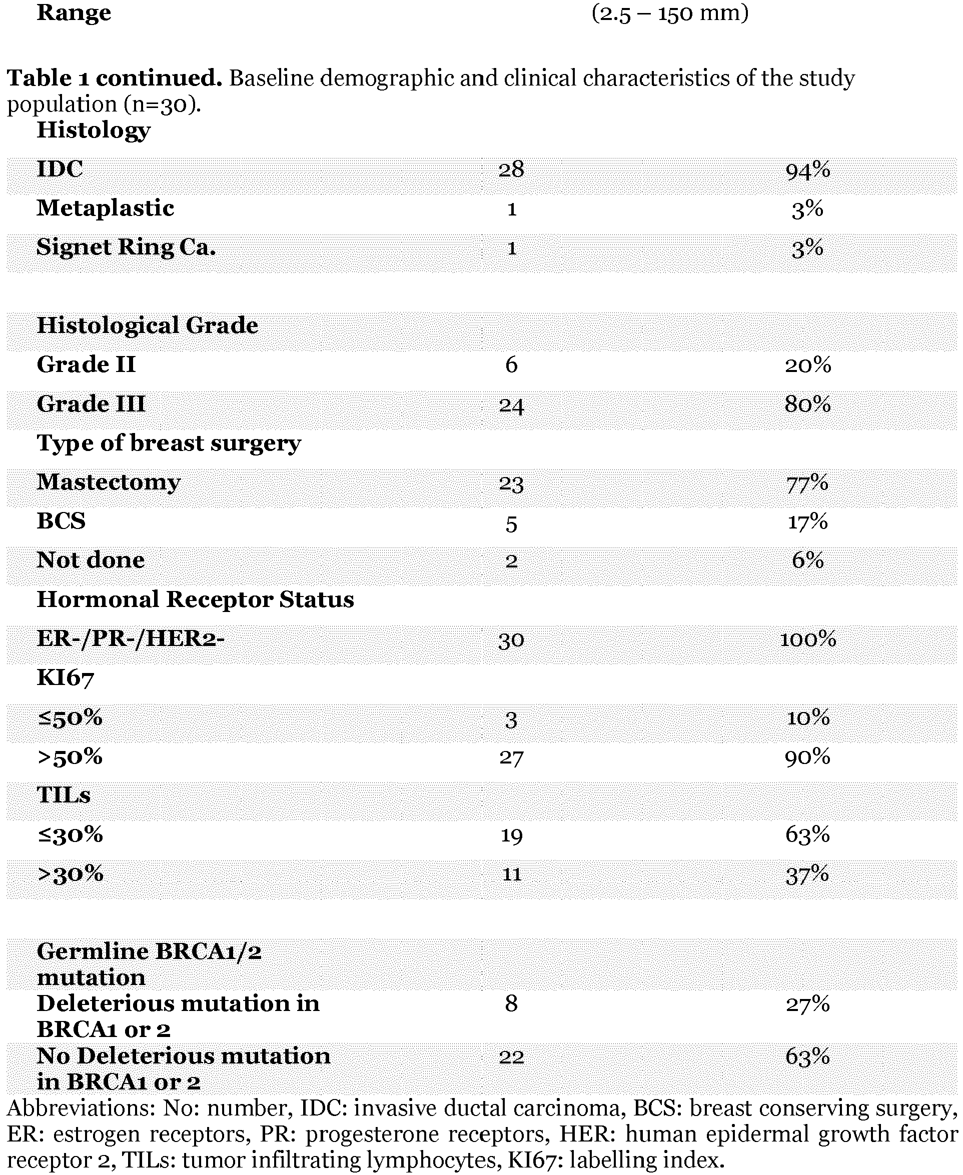

### Pathologic tumor response:

The primary end points were pathological complete response (pCR) in breast and axilla (ypTo/ypNo) and assessment for toxicity profiles. Patients were divided in classes depending on the residual cancer burden (RCB)

In the primary endpoint analysis, the overall pCR in residual cancer burden (RCB) class o and RCB class 0+1 were 66% and 69%, respectively (Table 2). pCR in axilla was achieved in 81% of patients (Table 2). Importantly, all patients (100%) with tumors bearing mutations in the BRCA1 or BRCA2 genes showed pCR (Table 2).

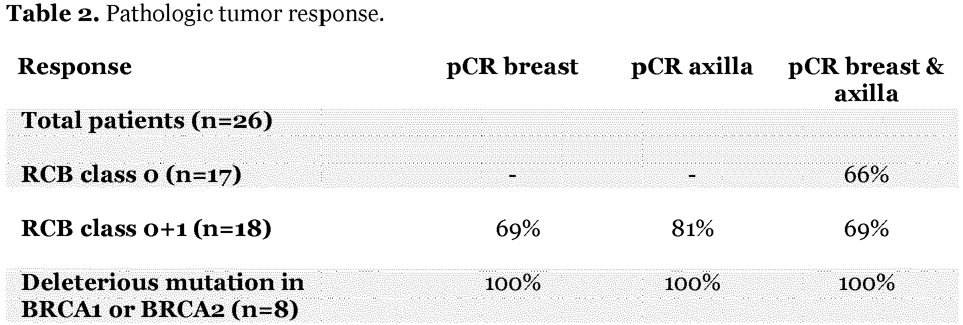

### Class RCB II (8 patients)

### Safety and tolerability:

All patients were assessable for toxicity; there were no treatment-related deaths following the study protocol. All cases of serious adverse events were improved or were resolved with appropriate treatments. The most common hematologic and non-hematologic toxicities are summarized in Table 3. Table 3 shows that most of the toxicities reported in this clinical trial were easily manageable, with no mortality reported among the treated patients.

**Table 3: Safety and tolerability.**

| **Types of Adverse Events** | **Grade 1 and 2** | **Grade 3 and 4** |
|---|---|---|
| Nausea | 15 | 1 |
| Vomiting | 7 | 2 |
| Diarrhea | 5 | 4 |
| mucositis | 3 | 1 |
| Fatigue | 13 | 3 |
| Neutropenia | 1 | 11 |
| Febrile neutropenia | 0 | 9 |
| Joint & muscle Pain | 8 | 1 |
| Peripheral neuropathy | 4 | 0 |
| AST/ALT increased | 4 | 3 |
| Abdominal Pain | 5 | 0 |
| Skin Rash | 3 | 0 |
| Infusion related reaction | 4 | 0 |
| Low Magnesium | 4 | 0 |
| Increase Lipid Profile | 7 | 0 |
| Renal impairment | 3 | 0 |

| | | |
|---|---|---|
| AST: Aspartate aminotransferase, ALT: Alanine aminotransferase | | |

### Conclusions:

This clinical trial shows that the sequential administration of tocilizumab followed by cisplatin/docetaxel is a highly efficient neoadjuvant treatment for locally advanced TNBC patients with unprecedented responses, especially in patients with tumors bearing BRCA1/2 mutations.

Of all patients, 66% showed an overall pCR in breast and axilla (=RCB class 0) and 69% can be attributed to RCB class 0 or class 1 (RCB class 0+1). Remarkably, pCR in axilla was reached in 81% of the patients. Strikingly, pCR in patients with tumors bearing mutations in the BRCA1 and BRCA2 genes was reached in 100% of the cases. This demonstrates that the novel combined application of an anti-interleukin-6 receptor antibody, a platinum-based antineoplastic drug, and a taxane of the present application exceeds the benefits of previous treatment options.

The present application shows that the pre-treatment with an anti-interleukin-6 receptor antibody, such as tocilizumab, effectively chemo-sensitizes cancer cells, leading to an excellent clinical response to treatment with a platinum-based antineoplastic drug/taxane, such as cisplatin/docetaxel, while also being safe and well-tolerated by the patients.

## Claims

1. A combination of an anti-interleukin-6 receptor antibody, a platinum-based antineoplastic drug, and a taxane, for use in a method of treatment of breast cancer.

2. The combination for use according to claim 1, wherein said anti-interleukin-6 receptor antibody is a humanized anti-interleukin-6 receptor antibody; preferably wherein said humanized anti-interleukin-6 receptor antibody is selected from: tocilizumab and a tocilizumab biosimilar; more preferably wherein said humanized anti-interleukin-6 receptor antibody is tocilizumab; and
wherein said platinum-based antineoplastic drug is selected from one or more of: cisplatin and carboplatin; preferably wherein said platinum-based antineoplastic drug is cisplatin; and
wherein said taxane is selected from one or more of: docetaxel, paclitaxel, and nab-paclitaxel; preferably wherein said taxane is docetaxel.

3. The combination for use according to any of the foregoing claims, wherein said combination is administered to a patient in need thereof; preferably wherein said patient is a mammal; more preferably wherein said patient is a human.

4. The combination for use according to any of the foregoing claims, wherein said combination is administered in a sequential manner, wherein said sequential manner comprises a dosing regimen selected from: administering a dose of said anti-interleukin-6 receptor antibody, followed by administering a dose of said platinum-based antineoplastic drug and a dose of said taxane; or administering a dose of said anti-interleukin-6 receptor antibody, followed by administering a dose of said platinum-based antineoplastic drug, followed by a dose of said taxane; or administering a dose of said anti-interleukin-6 receptor antibody, followed by administering a dose of said taxane, followed by a dose of said platinum-based antineoplastic drug;
or administering a dose of said platinum-based antineoplastic drug, followed by administering a dose of said anti-interleukin-6 receptor antibody and a dose of said taxane; or administering a dose of said platinum-based antineoplastic drug, followed by administering a dose of said anti-interleukin-6 receptor antibody, followed by a dose of said taxane; or administering a dose of said platinum-based antineoplastic drug, followed by administering a dose of said taxane, followed by a dose of said anti-interleukin-6 receptor antibody;
or administering a dose of said taxane, followed by administering a dose of said anti-interleukin-6 receptor antibody and a dose of said platinum-based antineoplastic drug; or administering a dose of said taxane, followed by administering a dose of said anti-interleukin-6 receptor antibody, followed by a dose of said platinum-based antineoplastic drug; or administering a dose of said taxane, followed by administering a dose of said platinum-based antineoplastic drug, followed by a dose of said anti-interleukin-6 receptor antibody;
preferably administering a dose of said anti-interleukin-6 receptor antibody, followed by administering a dose of said platinum-based antineoplastic drug and a dose of said taxane;
or wherein said combination is administered in a simultaneous manner, wherein said simultaneous manner comprises administering a dose of said anti-interleukin-6 receptor antibody, a dose of said platinum-based antineoplastic drug, and a dose of said taxane at the same time.

5. The combination for use according to any of the foregoing claims, wherein said method comprises
(1) administering a dose of said anti-interleukin-6 receptor antibody to a patient in need, on a first day of treatment;
(2) administering a dose of said platinum-based antineoplastic drug and a dose of said taxane to the patient in need, on days 2-8 of treatment, preferably on days 2-4 of treatment, more preferably on day 2 of treatment; and optionally
(3) administering a dose of said platinum-based antineoplastic drug and a dose of said taxane to the patient in need, in a periodical manner every 2-8 weeks, preferably every 2-6 weeks, more preferably every 4 weeks;
wherein said administering a dose of said platinum-based antineoplastic drug and a dose of said taxane of steps (2) and (3) is performed for a total of 4 to 7 cycles; preferably for a total of 6 cycles.

6. The combination for use according to any of the foregoing claims, wherein said anti-interleukin-6 receptor antibody is administered to said patient at a dosage of said anti-interleukin-6 receptor antibody in the range of 1 to 25 mg/kg; preferably in the range of 1 to 15 mg/kg; more preferably in the range of 1 to 12 mg/kg; most preferably in the range of 4 to 8 mg/kg.

7. The combination for use according to any of the foregoing claims, wherein said platinum-based antineoplastic drug is administered to said patient at a dosage of said platinum-based antineoplastic drug in the range of 1 to 250 mg/m²; preferably in the range of 1 to 180 mg/m²; more preferably in the range of 1 to 120 mg/m²; most preferably in the range of 30 to 60 mg/m².

8. The combination for use according to any of the foregoing claims, wherein said taxane is administered to said patient at a dosage of said taxane in the range of 1 to 250 mg/m²; preferably in the range of 1 to 180 mg/m²; more preferably in the range of 1 to 120 mg/m²; most preferably in the range of 30 to 60 mg/m².

9. The combination for use according to any of the foregoing claims, wherein said breast cancer is selected from: triple-negative breast cancer, locally advanced non-inflammatory breast cancer, invasive ductal carcinoma, inflammatory breast cancer, metastatic breast cancer, medullary carcinoma, tubular carcinoma, mucinous carcinoma, adenoid cystic carcinoma of the breast, metaplastic breast cancer, basal type breast cancer, or papillary breast cancer;
optionally wherein the cells of said breast cancer contain mutations in the BRCA1 and/or BRCA2 gene.

10. The combination for use according to any of the foregoing claims, wherein cells of said breast cancer are **characterized by** being estrogen receptor negative, or progesterone receptor negative, or human epidermal growth factor receptor 2 negative, or any possible combination thereof; preferably wherein cells of the cancer are **characterized by** a combination thereof; even more preferably wherein cells of the cancer are **characterized by** being estrogen receptor negative, progesterone receptor negative, and human epidermal growth factor receptor 2 negative, also called triple-negative breast cancer.

11. The combination for use according to any of the foregoing claims, wherein said anti-interleukin-6 receptor antibody is formulated and administered as an injection intravenously or subcutaneously;
wherein said platinum-based antineoplastic drug is formulated and administered as an injection intravenously;
wherein said taxane is formulated and administered as an injection intravenously.

12. The combination for use according to any of the foregoing claims, wherein said anti-interleukin-6 receptor antibody and/or said platinum-based antineoplastic drug and/or said taxane are administered in conjunction with further agent(s) or drug(s), such as granulocyte colony-stimulating factor.

13. The combination for use according to any of the foregoing claims, wherein said anti-interleukin-6 receptor antibody is tocilizumab, said platinum-based antineoplastic drug is cisplatin, and said taxane is docetaxel.

14. A kit for the preparation of an infusion solution for the administration of a combination of an anti-interleukin-6 receptor antibody, a platinum-based antineoplastic drug, and a taxane, as defined in any of the foregoing claims, said kit comprising a first container containing a composition comprising an anti-interleukin-6 receptor antibody, and a second container containing a composition comprising a pharmaceutically acceptable diluent; and/or
comprising a container containing a composition comprising a platinum-based antineoplastic drug, or a pharmaceutically acceptable salt thereof, and another container containing a composition comprising a pharmaceutically acceptable diluent; and/or
comprising a container containing a composition comprising a taxane, or a pharmaceutically acceptable salt thereof, and another container containing a composition comprising a pharmaceutically acceptable diluent;
wherein said containers are preferably selected from vials, bottles, pre-filled syringes, ampoules, infusion bags.

15. The kit according to claim 14, wherein the containers contain pre-mixed solutions selected from: said anti-interleukin-6 receptor antibody pre-mixed with said platinum-based antineoplastic drug, or a pharmaceutically acceptable salt thereof; said anti-interleukin-6 receptor antibody pre-mixed with said taxane, or a pharmaceutically acceptable salt thereof; said anti-interleukin-6 receptor antibody pre-mixed with said platinum-based antineoplastic drug, or a pharmaceutically acceptable salt thereof and said taxane, or a pharmaceutically acceptable salt thereof; and said platinum-based antineoplastic drug, or a pharmaceutically acceptable salt thereof pre-mixed with said taxane, or a pharmaceutically acceptable salt thereof.

16. The kit according to any of the claims 14-15, wherein said anti-interleukin-6 receptor antibody is provided as an injection concentrate;
wherein said platinum-based antineoplastic drug is provided in a form selected from: an injection concentrate, a pre-diluted solution, and a lyophilized powder for reconstitution;
wherein said taxane is provided in a form selected from: an injection concentrate, a pre-diluted solution, and a lyophilized powder for reconstitution.
